# EUROPÄISCHE PATENTANMELDUNG

(11) **EP 3 011 935 A1**
(43) Veröffentlichungstag der Anmeldung: **27.04.2016**
(21) Anmeldenummer: 15190944.7
(22) Anmeldetag: 22.10.2015
(51) Int. Cl.: A61F 2/50, A61F 7/00

(54) **WÄRME-/KÜHL-ELEMENT**

(30) Priorität: 22.10.2014 DE 102014221474
(71) Anmelder: Pohlig GmbH, 83278 Traunstein (DE)
(72) Erfinder: Pohlig, Kurt, 83278 Traunstein (DE); Schäfer, Michael, 83278 Traunstein (DE)
(74) Vertreter: Pfenning, Meinig & Partner mbB

(57) **Zusammenfassung**

Die Erfindung betrifft ein Wärme-/Kühl-Element zur gezielten Erwärmung und/oder Abkühlung eines Bereichs des menschlichen Körpers, wobei das Wärme-/Kühl-Element eine Wärmezu-/abführeinrichtung und ein mit der Wärmezu-/abführeinrichtung in wärmeleitendem Kontakt stehendes Kontaktelement mit einer Kontaktfläche aufweist, welche mit einem Oberflächenbereich des menschlichen Körpers kontaktierbar ist, so dass das Kontaktelement mit dem zu erwärmenden oder abzukühlenden Bereich in wärmeleitenden Kontakt bringbar ist, wobei eine Form der Kontaktfläche an die Form des Oberflächenbereichs und/oder die Form des zu erwärmenden oder abzukühlenden Bereichs angepasst ist.

## Beschreibung

Die vorliegende Erfindung bezieht sich auf ein Wärme-/Kühl-Element, das geeignet ist, durch Kontakt mit der Oberfläche eines menschlichen Körpers gezielt einen Bereich des menschlichen Körpers zu erwärmen oder abzukühlen. Die Erfindung betrifft auch eine Orthese oder Prothese mit einem Wärme-/Kühl-Element.

Eine gezielte Erwärmung oder Abkühlung kann die entsprechenden Bereiche des Körpers positiv beeinflussen. Beispielsweise kann eine schlechte Durchblutung in den Extremitäten von Patienten mit Orthesen oder in den Amputationsstümpfen von Patienten mit Prothesen zu Schmerzen in den Extremitäten oder Amputationsstümpfen führen, die durch eine gezielte Wärmezufuhr im Bereich von Oberflächen-nah verlaufenden Arterien oder Venen gelindert werden können.

Ferner ist es auch denkbar, dass eine gezielte Erwärmung der Oberfläche im Bereich des Kopfes durch Weitung von Oberflächen-nah verlaufenden Blutgefäßen eine schmerzlindernde Wirkung bei Migräne hat.

Des Weiteren kann auch eine zu starke Erwärmung der Extremitäten oder Amputationsstümpfe durch weitgehend geschlossene und schlecht belüftete Orthesen oder Prothesen, was ebenfalls zu einer Verringerung des Tragekomforts für die Patienten führen kann, durch eine gezielte Abkühlung von Bereichen der Extremitäten oder der Amputationsstümpfe vermieden werden. Andererseits ist bekannt, dass das Kühlen von entzündeten Körperpartien eine entzündungshemmende Wirkung hat.

Vermutlich beeinflussen Applikationen zur thermalen Energieübertragung die körpereigenen Sensoren, die wiederum die Menge des Blutzuflusses regulieren. Dabei wird bei Wärmestimulation der Blutzufluss erhöht. Bei Kälte verringert sich der Blutzufluss, sodass sich ein physiologischer Effekt einstellt.

Ferner kann nicht ausgeschlossen werden, dass die energiespendenden Elemente das Blut während der Diastole erwärmen (Herzmuskel ist entspannt, das Blut strömt nicht weiter, im Gegensatz zur Systole, wo das Blut mit hoher Fließgeschwindigkeit in die Peripherie gepumpt wird) und somit zur Erwärmung (oder zum Abkühlen) der Extremität beitragen.

Zur Erwärmung und/oder Abkühlung von Extremitäten oder Amputationsstümpfen innerhalb von Orthesen oder Prothesen können herkömmliche Mittel jedoch nicht verwendet werden, da diese nicht mit der Orthese oder Prothese kompatibel sind und es dadurch zu einer ungleichmäßigen Druckverteilung innerhalb des Schaftes der Orthese oder Prothese kommt. Außerdem ist die Effizienz der Wärmeübertragung gering.

Eine Aufgabe der vorliegenden Erfindung ist es daher, ein Wärme-/Kühl-Element bereitzustellen, das mit einer Orthese oder Prothese kompatibel und effizient in der Wärmeübertragung ist.

Die Aufgabe wird durch ein Wärme-/Kühl-Element nach Anspruch 1, eine Orthese oder Prothese nach Anspruch 12, ein Verfahren nach Anspruch 13 und Verwendungen nach Ansprüchen 14 und 15 gelöst. Vorteilhafte Weiter-bildungen des erfindungsgemäßen Wärme-/Kühl-Elements sind in den abhängigen Ansprüchen 2 bis 11 beschrieben.

Das erfindungsgemäße Wärme-/Kühl-Element zur Anordnung innerhalb einer Orthese oder Prothese zur gezielten Erwärmung und/oder Abkühlung eines Bereichs des menschlichen Körpers weist eine Wärmezu-/abführeinrichtung und ein mit der Wärmezu-/abführeinrichtung in wärmeleitendem Kontakt stehendes Kontaktelement mit einer Kontaktfläche auf, welche mit einem Oberflächenbereich des menschlichen Körpers kontaktierbar ist, so dass das Kontaktelement mit dem zu erwärmenden oder abzukühlenden Bereich in wärmeleitenden Kontakt bringbar ist.

Dieser wärmeleitende Kontakt zwischen der Kontaktfläche und dem zu erwärmenden oder abzukühlenden Bereich kann dabei direkt durch einen mechanischen Kontakt, z.B. zur Erwärmung oder Abkühlung der Haut selbst, oder indirekt über ein wärmeleitendes Medium, z.B. Haut und Körpergewebe, wenn z.B. Blutgefäße erwärmt oder abgekühlt werden sollen, hergestellt werden.

Erfindungsgemäß ist eine Form der Kontaktfläche an die Form des Oberflächenbereichs und/oder die Form des zu erwärmenden oder abzukühlenden Bereichs angepasst.

Dabei wird unter der Form des Oberflächenbereichs die Form des Oberflächenbereichs in allen drei Raumdimensionen, entlang der Oberfläche des menschlichen Körpers und senkrecht dazu, verstanden. Somit wird die Form des Oberflächenbereichs durch die Krümmung des Oberflächenbereichs und die Ausdehnung des Oberflächenbereichs entlang der Oberfläche bestimmt.

Die vorliegende Erfindung ermöglicht es zum einen die gesamte Kontaktfläche des Wärme-/Kühl-Elements zur gezielten Erwärmung oder Abkühlung des gewünschten Körperbereichs zu nutzen, was die Effizienz der Wärmeübertragung erhöht. Zum anderen ermöglicht die Erfindung eine Regulation der Temperatur von Extremitäten oder Amputationsstümpfen beim Tragen von Orthesen oder Prothesen und dadurch eine Verbesserung des Tragekomforts für den Patienten ohne die Funktion der Orthese oder Prothese zu beeinträchtigen.

Die Kontaktfläche kann dabei passend zum Oberflächenbereich veränderbar oder auch unveränderbar oder starr vorgeformt sein.

In einer bevorzugten Ausführungsform ist das erfindungsgemäße Wärme-/Kühl-Element derart ausgestaltet, dass das Kontaktelement in wärmeleitenden Kontakt mit einem nahe der Oberfläche des menschlichen Körpers verlaufenden Blutgefäß als zu erwärmenden oder abzukühlenden Bereich bringbar ist. Dies ist vorteilhaft, da zur Erwärmung oder Abkühlung von Oberflächennahen Blutgefäßen weniger Energie notwendig ist.

Das Blutgefäß kann eine Arterie oder eine Vene sein. Die Erwärmung oder Abkühlung von Arterien ist von Vorteil, wenn die Temperatur im Inneren des Körpers beeinflusst werden soll.

In einer weiteren vorteilhaften Ausgestaltung der Erfindung weist das Wärme-/Kühl-Element einen Temperatursensor, welcher wärme- oder Kühle-leitend mit dem Kontaktelement verbunden ist, zum Messen einer Temperatur und eine Temperatursteuereinheit, welche mit dem Temperatursensor und der Wärmezu-/abführeinrichtung verbunden ist, zum Steuern der Wärmezu-/abführeinrichtung basierend auf der gemessenen Temperatur auf. Dies ermöglicht eine präzise Einstellung einer vorbestimmten Solltemperatur sowie eine Nachjustierung der vorbestimmten Temperatur, falls eine äußere Umgebungstemperatur ändert.

In einer weiteren vorteilhaften Ausgestaltung weist das Wärme-/Kühl-Element ein Peltier-Element oder ein ähnliches Element auf. Insbesondere kann die Wärmezu-/abführeinrichtung ein Peltier-Element oder ein ähnliches Element sein.

Dabei kann das Wärme-/Kühl-Element selbst eine Stromversorgung für das Peltier-Element oder das ähnliche Element aufweisen oder das Peltier-Element oder das ähnliche Element kann mit einer externen Stromversorgung verbunden werden. Ersteres ist von Vorteil, wenn nur ein geringer Energiebedarf zum Betreiben des Peltier-Elements oder des ähnlichen Elements besteht.

Eine weitere Ausführungsform sieht vor, dass die Wärmezu-/abführeinrichtung zur Erwärmung und/oder Abkühlung eines Gases, insbesondere von Luft, ausgebildet ist, und dass das Kontaktelement ein Wärmetauscher ist, wobei die Wärmezu-/abführeinrichtung und der Wärmetauscher über das erwärmte oder abgekühlte Gas in wärmeleitendem Kontakt stehen.

Es ist auch denkbar, dass zwei verschiedene Wärme-/Kühl-Elemente zusammen verwendet werden, wobei diese miteinander in Wechselwirkung stehen. In diesem Fall kann die Wärmezu-/abführeinrichtung des ersten Wärme-/Kühl-Elements ein Kontaktelement eines zweiten Wärme-/Kühl-Elements sein, das mit dem Kontaktelement des ersten Wärme-/Kühl-Elements in wärmeleitendem Kontakt steht. Auf diese Weise könnte Wärme an einem nicht ausgekühlten Bereich des Körpers entzogen und als biologischer Transfer an einen aufzuwärmenden Bereich übertragen werden.

Das Wärme-/Kühl-Element kann derart ausgestaltet sein, dass der Wärmezu-/abführeinrichtung mittels einer externen Energiequelle Energie zuführbar oder entziehbar ist. Beispielsweise können Wärme-/Kälte-Pads vor der Verwendung durch eine externe Wärme- oder Kältequelle aufgeheizt bzw. abgekühlt werden.

Als externe Energiequelle kann auch Sonnenenergie genutzt werden.

Ferner können die Wärmezu-/abführeinrichtung und das Kontaktelement als ein gemeinsames Bauteil ausgebildet sein. Die Wärmezu-/abführeinrichtung kann dabei auch in das Kontaktelement integriert sein.

Vorteilhafterweise enthält das Kontaktelement Metall, Aluminium, ein Gel-Pad, ein Wärme-/Kühl-Gelkissen, Wärme-/Kälte-Pad enthält oder besteht daraus. Der Vorteil an Gel-Pads, Wärme-/Kühl-Gelkissen und Wärme-/Kälte-Pads besteht darin, dass sich diese gut der Oberfläche des Körpers anpassen und dadurch kein unangenehmes Druckempfinden für den Patienten verursachen. Es können auch solche Wärme-Pads verwendet werden, die aufgrund einer chemischen Reaktion für eine längere Zeit Wärme abgeben.

Die vorliegende Erfindung schließt auch eine Orthese oder Prothese ein, die ein oder mehrere erfindungsgemäße Wärme-/Kühl-Elemente aufweist.

Des Weiteren umfasst die vorliegende Erfindung ein Verfahren zur gezielten Erwärmung oder Abkühlung eines Bereichs des menschlichen Körpers mit mindestens einem erfindungsgemäßen Wärme-/Kühl-Element. Gemäß dem Verfahren wird die Kontaktfläche mit einem Oberflächenbereich des menschlichen Körpers kontaktiert, so dass das Kontaktelement mit dem zu erwärmenden oder abzukühlenden Bereich in wärmeleitenden Kontakt gebracht und Wärme über die Wärmezu-/abführeinrichtung dem Kontaktelement zu- oder von dem Kontaktelement abgeführt wird.

Ferner ist eine Verwendung eines erfindungsgemäßen Verfahrens zur Regulation der Temperatur eines Körperteils, das von einer Orthese oder Prothese aufgenommenen wird, also sich innerhalb einer erfindungsgemäßen Orthese oder Prothese befindet, vorgesehen. In diesem Fall können ein oder mehrere erfindungsgemäße Wärme-/Kühl-Elemente derart innerhalb der Orthese oder Prothese angeordnet sein, dass die Kontaktfläche mit einem Oberflächenbereich des von der Orthese oder Prothese aufgenommenen Körperteils, also z.B. eines Amputationsstumpfes, kontaktiert wird, so dass das Kontaktelement mit dem zu erwärmenden oder abzukühlenden Bereich des Amputationsstumpfes in wärmeleitenden Kontakt gebracht und Wärme über die Wärmezu-/abführeinrichtung dem Kontaktelement zu- oder von dem Kontaktelement abgeführt wird. Über das Kontaktelement wiederum wird dann dem zu erwärmenden oder abzukühlenden Bereich Wärme zugeführt oder von diesem abgeführt.

Das erfindungsgemäße Verfahren kann auch zur Schmerztherapie, vorzugsweise zur Therapie von Migräne oder Phantomschmerzen eingesetzt werden.

Im Folgenden werden einige Beispiele eines erfindungsgemäßen Wärme-/Kühl-Elements sowie einer erfindungsgemäßen Orthese oder Prothese anhand von Figuren beschrieben. Dabei werden verschiedene erfindungswesentliche oder vorteilhafte Elemente im Rahmen dieser Beispiele genannt, wobei auch einzelne dieser Elemente als solche zur Weiterbildung der Erfindung - auch herausgelöst aus dem Kontext der Beispiele - verwendet werden können. Ferner entsprechen in den Figuren gezeigte gleiche oder ähnliche Elemente gleichen oder ähnlichen Bezugszeichen.

Es zeigen
- Figur 1: ein erfindungsgemäßes Wärme-/Kühl-Element,
- Figur 2: einen Beinstumpf, an dem ein erfindungsgemäßes Wärme-/Kühl-Element angeordnet ist, und
- Figur 3: einen den Beinstumpf aus Figur 2 umschließenden Prothesenschaft mit einem integrierten erfindungsgemäßen Wärme-/Kühl-Element in einer Schnittansicht.

In den Figuren werden die folgenden Bezugszeichen verwendet:
- 1: Wärme-/Kühl-Element
- 2: Wärmezu-/abführeinrichtung
- 2a: Obere Hauptfläche
- 3: Kontaktelement
- 3a: Kurze Seite
- 3b: Halbkreisförmige Seite
- 4: Kontaktfläche
- 5: Temperatursensor
- 6: Temperatursteuereinheit
- 7: Verbindungskabel
- 8: Sensorkabel
- 9: Beinstumpf
- 10: Prothesenschaft
- 11: Außenseite des Beinstumpfs

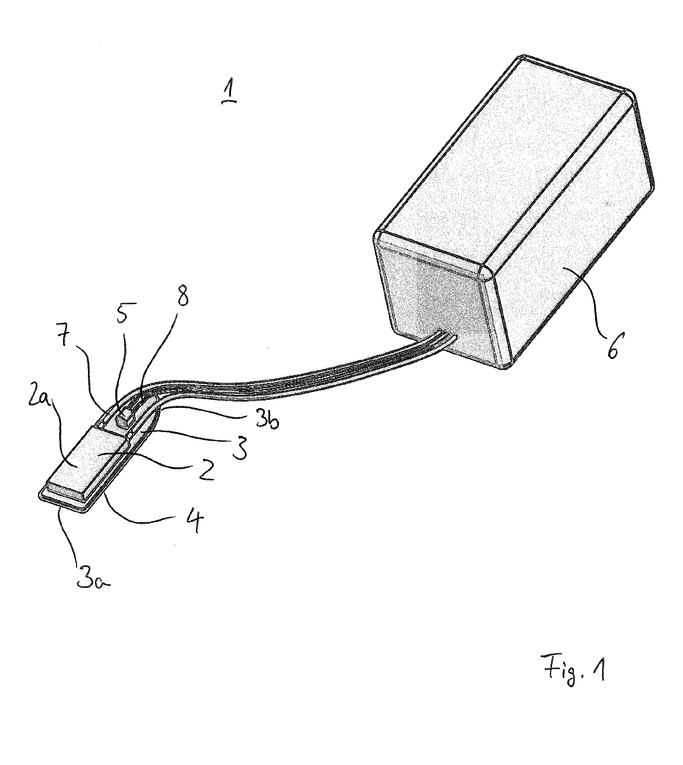

Figur 1 zeigt ein erfindungsgemäßes Wärme-/Kühl-Element 1. Das Wärme-/Kühl-Element umfasst eine Wärmezu-/abführeinrichtung 2, die eine flache Quaderform mit einer oberen Hauptfläche 2a und einer zu der oberen Hauptfläche 2a parallelen, unteren Hauptfläche (in Figur 1 nicht dargestellt) aufweist. Bei der Wärmezu-/abführeinrichtung 2 kann es sich beispielsweise um ein Peltier-Element handeln. Entlang der unteren Hauptfläche ist die Wärmezu-/abführeinrichtung 2 auf einem Kontaktelement 3 befestigt. Das Kontaktelement 3 weist in etwa eine plattenartige, rechteckige Form mit zwei gegenüberliegenden langen Seiten und einer kurzen Seite 3a auf, wobei eine der kurzen Seite 3a gegenüberliegende Seite 3b halbkreisförmig gebogen ausgestaltet ist. Das Kontaktelement 3 weist eine etwas größere Grundfläche als die Wärmezu-/abführeinrichtung 2 auf, wobei die Wärmezu-/abführeinrichtung 2 bezüglich der kurzen Seite 3a des Kontaktelementes 3 mittig und bezüglich der langen Seiten des Kontaktelementes 3 auf der der halbkreisförmigen Seite 3b abgewandten Seite des Kontaktelementes 3 platziert ist. Die flache, längliche Form des Kontaktelementes 3 ermöglicht eine optimale Platzierung des Kontaktelementes 3 auf der Haut entlang einer Arterie. Das Kontaktelement 3 weist an einer der Wärmezu-/abführeinrichtung 2 abgewandten Seite eine Kontaktfläche 4 auf. Durch die länglich geformte Kontaktfläche 4 kann ein durch eine mit der Kontaktfläche 4 in wärmeleitendem Kontakt stehende Arterie fließendes Blut entlang einer möglichst großen Fläche schnell erwärmt bzw. abgekühlt werden.

Die Wärmezu/abführeinrichtung 2 ist mit einer Temperatursteuereinheit 6 über Verbindungskabel 7 elektrisch verbunden. Die Temperatursteuereinheit 6 weist ein quaderförmiges Gehäuse auf, in dem eine Stromversorgung wie z.B. eine Batterie oder ein Akkumulator, und eine Steuerungselektronik untergebracht sind (Stromversorgung und Steuerungselektronik sind in Figur 1 nicht dargestellt). Über die Verbindungskabel 7 wird die Wärmezu-/abführeinrichtung 2 zur Erzeugung von Wärme bzw. zum Abführen von Wärme von der Temperatursteuereinheit 6 mit Strom versorgt. Im Falle eines Peltier-Elementes als Wärmezu-/abführeinrichtung 2 ist ein erstes Verbindungskabel 7 mit einer im Bereich der oberen Hauptfläche 2a gelegenen Oberseite des Peltier-Elementes verbunden und ein zweites Verbindungskabel 7 mit einer im Bereich der unteren Hauptfläche gelegenen Unterseite des Peltier-Elementes verbunden. Hieraus resultiert eine Potentialdifferenz zwischen der Ober- und der Unterseite, was zu einer Temperaturdifferenz zwischen der Ober- und der Unterseite führt. Aufgrund dieser Temperaturdifferenz wird das mit dem Peltier-Element in wärmeleitendem Kontakt stehende Kontaktelement 3 erwärmt bzw. abgekühlt, welches wiederum ein mit dem Kontaktelement 3 in wärmeleitendem Kontakt stehenden Körperbereich erwärmt bzw. abkühlt.

Auf dem Kontaktelement 3 ist ferner benachbart zur Wärmezu-/abführeinrichtung 2 in einem der halbkreisförmigen Seite 3b zugewandten Bereich des Kontaktelementes 3 ein Temperatursensor 5 angeordnet. Dieser Temperatursensor 5 steht in wärmeleitendem Kontakt mit dem Kontaktelement 3. Der Temperatursensor 5 ist mit der Temperatursteuereinheit 6 über Sensorkabel 8 elektrisch verbunden. Mittels des Temperatursensors 5 kann eine Temperatur des Kontaktelementes 3 gemessen werden. Die gemessenen Temperaturmesswerte können dann an die Steuerungselektronik in der Temperatursteuereinheit 6 über die Sensorkabel 8 übertragen werden. Basierend auf den gemessenen Temperaturmesswerten kann dann eine von dem Kontaktelement 3 abgegebene bzw. abzuführende Wärme gesteuert werde. Ferner wird der Temperatursensor 5 über die Sensorkabel 8 mit Strom versorgt.

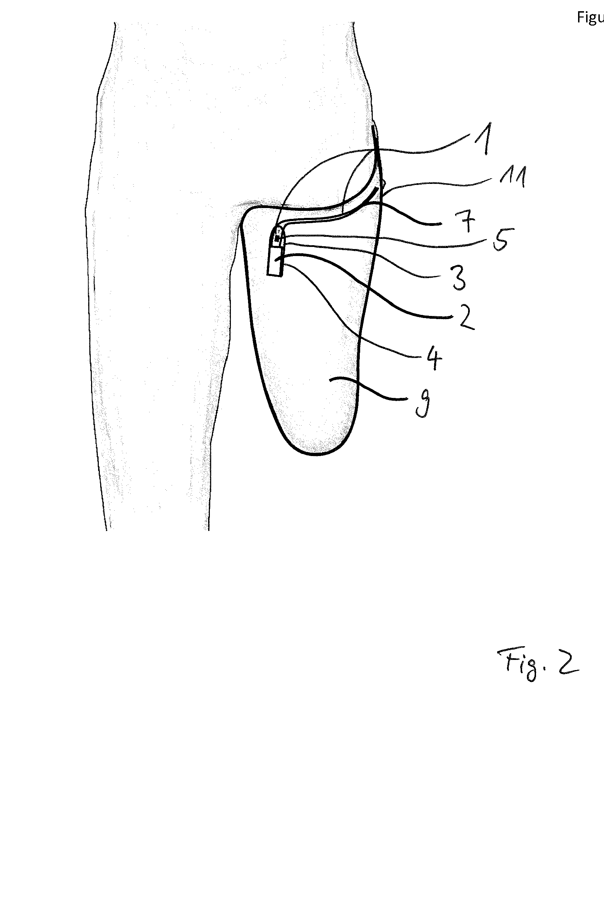

Figur 2 zeigt einen Beinstumpf 9, beispielsweise aufgrund einer Unterschenkelamputation, und ein zur Körpermitte hin an diesen Beinstumpf 9 angeordnetes erfindungsgemäßes Wärme-/Kühl-Element 1 wie es in Figur 1 beschrieben worden ist. Das Wärme-/Kühl-Element 1 ist mit seinem Kontaktelement 3 entlang einer Oberschenkelarterie (Arteria Femuralis) an einer Stelle, an der die Oberschenkelarterie Hautoberflächen-nah verläuft, angeordnet. Die mit der Wärmezu-/abführeinrichtung 2 verbundenen Verbindungskabel 7 verlaufen entlang eines oberen Endes des Beinstumpfs 9 zu einer Außenseite 11 des Beinstumpfs 9. Die mit dem Temperatursensor 5 verbundenen Sensorkabel sowie die Temperatursteuereinheit 6 sind in Figur 2 nicht dargestellt.

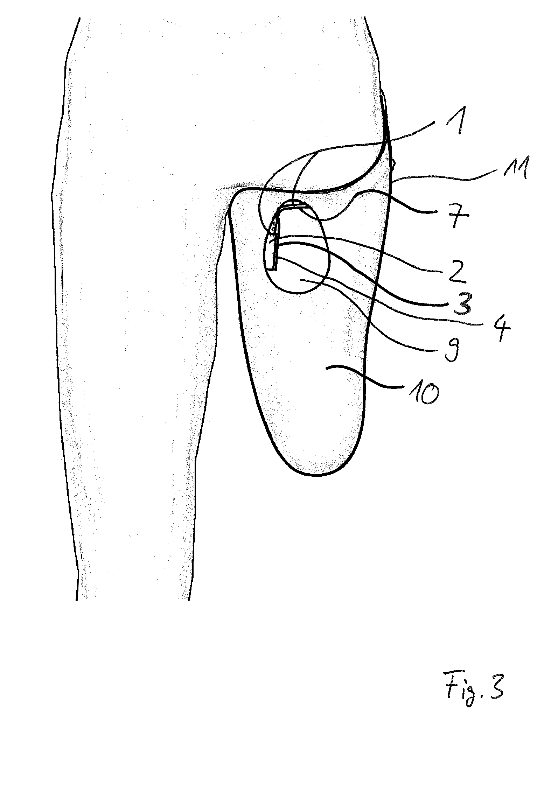

Figur 3 zeigt den Beinstumpf 9 aus Figur 2, wobei in Figur 3 ein Prothesenschaft 10 über den Beinstumpf 9 gezogen ist. Auf zum Beinstumpf 9 zugewandten Innenseite des Prothesenschafts 10 ist ein wie in Figur 1 dargestelltes Wärme-/Kühl-Element 1 integriert, das wie in Figur 2 entlang der Oberschenkelarterie (Arteria Femuralis), an einer Stelle, an welcher die Oberschenkelarterie Hautoberflächen-nah verläuft, angeordnet ist. Zur besseren Sichtbarkeit der Position des erfindungsgemäßen Wärme-/Kühl-Elementes 1 ist die Darstellung des Prothesenschafts 10 im Bereich des Wärme-/Kühl-Elementes 1 oval ausgeschnitten. Die mit der Wärmezu-/abführeinrichtung 2 verbundenen Verbindungskabel 7 des Wärme-/Kühl-Elementes 1 verlaufen in einem oberen Bereich des Beinstumpfes in der Nähe des Hüftgelenks zu einer Außenseite 11 des Beinstumpfs 9 hin.

## Patentansprüche

1. Wärme-/Kühl-Element zur Anordnung innerhalb einer Prothese oder Orthese zur gezielten Erwärmung und/oder Abkühlung eines Bereichs des menschlichen Körpers, wobei das Wärme-/Kühl-Element eine Wärmezu-/abführeinrichtung und ein mit der Wärmezu-/abführeinrichtung in wärmeleitendem Kontakt stehendes Kontaktelement mit einer Kontaktfläche aufweist, welche mit einem Oberflächenbereich des menschlichen Körpers kontaktierbar ist, so dass das Kontaktelement mit dem zu erwärmenden oder abzukühlenden Bereich in wärmeleitenden Kontakt bringbar ist, wobei eine Form der Kontaktfläche an die Form des Oberflächenbereichs und/oder die Form des zu erwärmenden oder abzukühlenden Bereichs angepasst ist.

2. Wärme-/Kühl-Element nach dem vorhergehenden Anspruch, **dadurch gekennzeichnet, dass** der zu erwärmende oder abzukühlende Bereich ein nahe der Oberfläche des menschlichen Körpers verlaufendes Blutgefäß ist und das Kontaktelement in wärmeleitenden Kontakt mit dem Blutgefäß bringbar ist.

3. Wärme-/Kühl-Element nach dem vorhergehenden Anspruch, **dadurch gekennzeichnet, dass** das Blutgefäß eine Arterie oder eine Vene ist.

4. Wärme-/Kühl-Element nach einem der vorhergehenden Ansprüche, **dadurch gekennzeichnet, dass** das Wärme-/Kühl-Element einen Temperatursensor, welcher wärme- oder Kühle-leitend mit dem Kontaktelement verbunden ist, zum Messen einer Temperatur und eine Temperatursteuereinheit, welche mit dem Temperatursensor und der Wärmezu-/abführeinrichtung verbunden ist, zum Steuern der Wärmezu-/abführeinrichtung basierend auf der gemessenen Temperatur aufweist.

5. Wärme-/Kühl-Element nach einem der vorhergehenden Ansprüche, **dadurch gekennzeichnet, dass** das Wärme-/Kühl-Element ein Peltier-Element oder ein ähnliches Element aufweist.

6. Wärme-/Kühl-Element nach dem vorhergehenden Anspruch, **dadurch gekennzeichnet, dass** das Wärme-/Kühl-Element eine Stromversorgung für das Peltier-Element oder das ähnliche Element aufweist.

7. Wärme-/Kühl-Element nach einem der vorhergehenden Ansprüche, **dadurch gekennzeichnet, dass** die Wärmezu-/abführeinrichtung zur Erwärmung und/oder Abkühlung eines Gases, insbesondere von Luft, ausgebildet ist, und dass das Kontaktelement ein Wärmetauscher ist, wobei die Wärmezu-/abführeinrichtung und der Wärmetauscher über das erwärmte oder abgekühlte Gas in wärmeleitendem Kontakt stehen.

8. Wärme-/Kühl-Element nach einem der vorhergehenden Ansprüche, **dadurch gekennzeichnet, dass** die Wärmezu-/abführeinrichtung ein Kontaktelement eines zweiten Wärme-/Kühl-Elements ist, das mit dem Kontaktelement des ersten Wärme-/Kühl-Elements in wärmeleitendem Kontakt steht.

9. Wärme-/Kühl-Element nach einem der vorhergehenden Ansprüche, **dadurch gekennzeichnet, dass** der Wärmezu-/abführeinrichtung mittels einer externen Energiequelle Energie zuführbar oder entziehbar ist.

10. Wärme-/Kühl-Element nach einem der vorhergehenden Ansprüche, **dadurch gekennzeichnet, dass** die Wärmezu-/abführeinrichtung und das Kontaktelement als ein gemeinsames Bauteil ausgebildet sind oder dass die Wärmezu-/abführeinrichtung in das Kontaktelement integriert ist.

11. Wärme-/Kühl-Element nach einem der vorhergehenden Ansprüche, **dadurch gekennzeichnet, dass** das Kontaktelement Metall, Aluminium, ein Gel-Pad, ein Wärme-/Kühl-Gelkissen, Wärme-/Kälte-Pad enthält oder daraus besteht.

12. Orthese oder Prothese **dadurch gekennzeichnet, dass** die Orthese oder Prothese ein oder mehrere Wärme-/Kühl-Elemente nach einem der vorhergehenden Ansprüche aufweist.

13. Verfahren zur gezielten Erwärmung oder Abkühlung eines Bereichs des menschlichen Körpers mit mindestens einem Wärme-/Kühl-Element nach einem der Ansprüche 1 bis 11, wobei die Kontaktfläche mit einem Oberflächenbereich des menschlichen Körpers kontaktiert wird, so dass das Kontaktelement mit dem zu erwärmenden oder abzukühlenden Bereich in wärmeleitenden Kontakt gebracht wird und Wärme über die Wärmezu-/abführeinrichtung dem Kontaktelement zu- oder von dem Kontaktelement abgeführt wird.

14. Verwendung eines Verfahrens nach dem vorhergehenden Anspruch zur Regulation der Temperatur eines von einer Orthese oder Prothese nach Anspruch 12 aufgenommenen Körperteils.

15. Verwendung eines Verfahrens nach Anspruch 13 zur Schmerztherapie, vorzugsweise zur Therapie von Migräne oder Phantomschmerzen.
